# EUROPÄISCHE PATENTSCHRIFT

(11) **EP 3 346 958 B1**
(45) Veröffentlichungstag und Bekanntmachung des Hinweises auf die Patenterteilung: **03.07.2019**
(21) Anmeldenummer: 16751587.3
(22) Anmeldetag: 15.08.2016
(51) Int. Cl.: A61F 5/01

(54) **SCHWENKLIMITIERBARES BIAXIALES GELENK**
PIVOTING-LIMITABLE BIAXIAL JOINT
ARTICULATION BIAXIALE DONT LE PIVOTEMENT PEUT ÊTRE LIMITÉ

(30) Priorität: 09.09.2015 DE 102015217205
(43) Veröffentlichungstag der Anmeldung: 18.07.2018
(73) Patentinhaber: Bauerfeind AG, 07937 Zeulenroda-Triebes (DE)
(72) Erfinder: GÖRNERT, Florian, 07937 Zeulenroda-Triebes (DE); HEBENSTREIT, Sandro, 07937 Zeulenroda-Triebes (DE)
(74) Vertreter: Schwahn, Hartmut
(86) Internationale Anmeldenummer: PCT/EP2016/069321
(87) Internationale Veröffentlichungsnummer: WO 2017/041993

(56) Entgegenhaltungen:
- WO-A1-01/10360
- US-A- 5 443 444
- US-A1- 2006 287 624

## Beschreibung

Die vorliegende Erfindung betrifft ein schwenklimitierbares biaxiales Gelenk, besonders für eine Gelenkorthese zur Unterstützung und variablen Limitierung der Gelenkbeugung. Die Erfindung stellt eine Verbesserung bekannter Gelenkorthesen bereit.

Medizinische Hilfsmittel für die Orthopädie, sogenannte Orthesen, zur Aufrechterhaltung, Stabilisierung oder Wiederherstellung einer Gelenkfunktion sind bekannt. Zur therapeutischen mechanischen Unterstützung der Gelenkfunktion, besonders zur kontrollierten Führung der Gelenkbewegung, vor allem zur therapeutisch häufig erforderlichen Limitierung des Beugewinkels des Gelenks sind mechanisch stabile Orthesen, sogenannte "Hartrahmen-Orthesen" bekannt. Diese setzen mechanisch beiderseits des Gelenks an und überbrücken das Gelenk mit einem steifen Rahmen oder Brücke. Die Rahmenkonstruktion der Orthese selbst ist dazu zumindest im Bereich des Körpergelenks schwenkbar und kann so die Gelenkfunktion des Körpergelenks mechanisch unterstützen, führen oder begrenzen.

Es hat sich herausgestellt, dass Körpergelenke mechanisch nicht durch eine einfache monoaxiale "Scharnierfunktion" nachbildbar sind. Um nachteilige Störkräfte oder Verspannungen an solchen Gelenkorthesen zu vermeiden, wurden sogenannte biaxiale oder multiaxiale Gelenke entwickelt, die eine anatomisch korrektere Verschwenkung der Gelenkorthesenteile in zumindest zwei benachbarten Gelenkachsen zulassen. Obere und untere, das heißt distale und proximale Abschnitte einer Gelenkorthese werden daher mit Gelenkschienen, die ein biaxiales Zentralgelenk in einem Gehäuse aufweisen, verbunden. Um die Verschwenkung der Gelenkschienen in dem biaxialen Gelenk mechanisch zweckmäßig umzusetzen, sind die beiden in dem Gelenk schwenkbaren Gelenkschienen bekanntermaßen über gezahnte Gelenkköpfe, die miteinander kämmen, geführt.

Insbesondere für therapeutische Maßnahmen ist es wünschenswert, die Schwenkbewegung dieses biaxialen Gelenks an der Gelenkorthese - und damit die Beugung und/oder Streckung des Körpergelenks selbst - zumindest zeitweise zu begrenzen. Der Stand der Technik kennt hier verschiedene Maßnahmen, die jedoch entweder mechanisch unzureichend sind oder in der Bedienung so komplex, sodass sich aufgrund von Bedienungsfehlern die gewünschte Funktion oft nicht einstellt. Der vorliegenden Erfindung lag die Aufgabe zugrunde, ein an sich bekanntes biaxiales Gelenk an einer Gelenkschiene, geeignet zur Verwendung an einer Gelenkorthese, spezifisch an einer Hartrahmen-Orthese, so zu verbessern, dass der Schwenkwinkel dieses Gelenks mit einfachen Mitteln zuverlässig und zumindest zeitweise begrenzbar ist. Besonders soll die Schwenklimitierung einfach anwendbar und gleichzeitig mechanisch zuverlässig und robust ausgeführt sein. Zusätzlich soll die Möglichkeit bestehen, auf einfache Weise, die Schwenklimitierung in verschiedenen Stufen bis hin zur Totalblockade der Gelenkfunktion umzusetzen.

US 2006/287624 A1 offenbart ein biaxiales Gelenk für eine Gelenkorthese.

Zur Lösung stellt die Erfindung den Gegenstand nach Hauptanspruch 1 bereit, besonders ein biaxiales Gelenk für eine Gelenkorthese, enthaltend Gelenkschienen mit jeweils gezahnten Gelenkköpfen, die in einem Gelenkgehäuse zwischen einer äußeren und einer inneren Gehäuseplatte geführt sind und an Gelenkachsen in dem Gelenkgehäuse gelagert sind, wobei die Gelenkschienen an ihren Gelenkköpfen jeweils miteinander kämmend gemeinsam um die Gelenkachsen in dem Gelenkgehäuse verschwenkbar sind, wobei erfindungsgemäß mindestens eine der Gehäuseplatten, insbesondere mindestens die äußere Gehäuseplatte, mindestens eine in der Fläche der Gehäuseplatte liegende Durchbrechung aufweist, worin jeweils ein Limitierungskeil in axialer Richtung in das Gehäuse eingesetzt werden kann. Erfindungsgemäß erlaubt der dort axial eingesetzte Limitierungskeil im eingesetzten Zustand, dass die Verschwenkung der Gelenkschienen in dem Gelenkgehäuse in zumindest einer Schwenkrichtung begrenzt ist, da vorgesehen ist, dass der Limitierungskeil mit korrespondierenden Schultern der Gelencköpfe ab einem bestimmten Verschwenkwinkel der Gelenkschienen in Eingriff kommt.

Bevorzugt ist vorgesehen, dass der Limitierungskeil sich in axialer Richtung durch das gesamte Gelenkgehäuse erstreckt und insbesondere in beiden, äußerer und innerer Gehäuseplatte des Gelenkgehäuses, in einer jeweiligen Durchbrechung kraftschlüssig, besonders formschlüssig und in besonderer Ausgestaltung ausschließlich oder zusätzlich reibschlüssig, aufgrund von Passung, geführt wird. Demgemäß ist in bevorzugter Ausführung des biaxialen Gelenks vorgesehen, dass beide Gehäuseplatten jeweils eine Durchbrechung zur Aufnahme und Führung dieses Limitierungskeils in axialer Richtung aufweisen.

Besonders ist vorgesehen, dass der bevorzugt einstückig ausgebildete Limitierungskeil in zumindest drei funktionale und räumlichkörperliche Abschnitte einteilbar ist. Eine äußere Führungsschulter ist vorgesehen, die mit oder in der Durchbrechung der äußeren Gehäuseplatte in Eingriff zu kommen, eine innere Führungsschulter ist vorgesehen, mit oder in der Durchbrechung der inneren Gehäuseplatte in Eingriff zu kommen. Ein dazwischen liegender zentraler Limitierungsabschnitt weist Anschlagsflächen auf, woran korrespondierende Schultern der Gelenkköpfe der Gelenkschienen bei deren Verschwenken anschlagen können, um die Verschwenkung der Gelenkschienen an dem Gelenk zu begrenzen.

Bevorzugt ist der Limitierungskeil einstückig und insbesondere einteilig ausgebildet. Der Limitierungskeil ist vorzugsweise aus schlagzähem Material, besonders bevorzugt hochfestes Polymer oder Metall, bevorzugt ausgewählt aus Aluminium, Magnesium, Titan oder diese enthaltende Legierungen, gefertigt. In einer besonderen Ausgestaltung ist der Limitierungskeil aus einem elastisch verformbaren Material gebildet, dessen Elastizität und temporäre Verformbarkeit eine gedämpfte Begrenzung der Verschwenkung der Gelenkschienen ermöglicht. Diese Ausgestaltung des Limitierungskeils aus einem elastischen Werkstoff, zumindest im Bereich des zentralen Limitierungsabschnitts des Limitierungskeils, besonders zumindest im Bereich der Anschlagsflächen des zentralen Limitierungsabschnitts, ermöglicht vorteilhaft ein physiologisch und therapeutisch zweckmäßiges "Einbremsen" in die Limitierung der Gelenkbewegung, was zum einen dem Träger frühzeitig die Limitierung signalisiert, zum anderen ansonsten gegebenenfalls abrupt einsetzende Gegenkräfte, die auch zu einer Überlastung des Gelenks als solches führen können, verhindern. Die elastischen Eigenschaften des Limitierungskeils beziehungsweise der maßgeblichen Abschnitte davon können durch geeignete elastische Werkstoffe wie Elastomere oder alternativ und zusätzlich durch Federelemente, Flachfedern, Schraubenfedern, umgesetzt sein.

Die Erfindung erlaubt gegenüber schwenklimitierten wie axialen Gelenken des Standes der Technik vorteilhafterweise eine leicht bedienbare und mechanisch äußerst stabile Begrenzung der Schwenkbewegung der Gelenkschienen. Durch die neuartige Lagerung des mindestens einen Limitierungskeils in der mindestens einen, bevorzugt in beiden Gehäuseplatten, wobei sich der Limitierungskeil in axialer Richtung durch das Gehäuse erstreckt, kann eine mechanisch besonders stabile Limitierung oder Blockade der Schwenkbewegung der Gelenkschienen über den Anschlag von deren Gelenkköpfe an den eingesetzten Limitierungskeil erzielt werden. Da die Gelenkschienen in ihren Gelenkköpfen in axialer Richtung in den Schwenkachsen gefasst sind, und der Limitierungskeil ebenfalls in axialer Richtung eingesetzt und durch das Gehäuse gefasst ist, werden sämtliche zum Zeitpunkt der Schwenklimitierung beim Anschlag der Gelenkköpfe an den Limitierungskeil auftretenden Querkräfte gut und sicher aufgenommen, ohne dass es zu resultierenden Kraftvektoren kommen könnte, welche den sicheren Halt des Limitierungskeils in dem Gelenkgehäuse entgegenstehen. Im Gegenteil, durch die beim Gelenkanschlag resultierenden Kräfte, wird der Gelenkkeil aufgrund von Form- und Reibschluss noch sicherer in dem Gelenkgehäuse gehalten als in seinem unbelasteten Zustand. Vorteilhafterweise ist der Limitierungskeil aber gerade in unbelastetem Zustand, indem er nicht schwenklimitierend wirkt, leicht und vorzugsweise ohne weitere Maßnahmen aus dem Gelenkgehäuse herausnehmbar, was die Bedienung erleichtert.

Erfindungsgemäß ist der Limitierungskeil mit mindestens einer, besonders aber mit zwei gegenüberliegenden dezidierten Anschlagflächen oder Anschlagschultern versehen, die zur Schwenklimitierung mit dezidierten Schultern oder Vorsprüngen an den Gelenkköpfen in Kontakt kommen können und die Schwenkbewegung dadurch stoppen können. Einfache Stifte, besonders mit kreisrundem Querschnitt, die in ein Gelenkgehäuse eingesteckt werden können und dort insbesondere in Löchern oder Langlöchern der schwenkenden Gelenkschienen oder deren Gelenkköpfe in Eingriff kommen, um insbesondere die Gelenkbewegung zu blockieren, sind ausgeschlossen. Ein erfindungsgemäßer Limitierungskeil ist kein Blockierstift. Ohne an die Theorie gebunden sein zu wollen, ist festzustellen, dass solche einfachen Blockierstifte zwar eine Gelenkschiene insgesamt blockieren können, diese aber in der Praxis bei der Funktion der Schwenklimitierung aus der Bewegung heraus zu hohen mechanischen Belastungen ausgesetzt sind. Demgegenüber erlaubt die erfindungsmäßige Konstruktion eines spezifisch geformten Limitierungskeils mit Schultern und dezidierten Anschlagflächen in der praktischen Anwendung sogar eine mechanische Selbststabilisierung der Gesamtgelenkanordnung im Limitierungsfall (Anschlag), auch wenn dieser aus der vollen Bewegung heraus eintritt.

In bevorzugten Ausgestaltungen sieht die Erfindung zusätzlich vor, dass Limitierungskeile verschiedener Abmessungen zumindest des mittleren Limitierungsabschnitts des Limitierungskeils wahlweise in das Gelenkgehäuse einsetzbar sind, um den Grad der Schwenklimitierung des Gelenks frei wählbar zu machen. Nach Art eines Baukastensystems können so dem Bediener der Gelenkorthese verschiedene und einfach austauschbare Limitierungskeile zur Verfügung gestellt werden, um indikationsbedingt, oder je nach Therapiephase den Grad der Schwenklimitierung mehr oder weniger zu beschränken. Es kann so eine einfach anwendbare und mechanisch stabilere Anpassung der Schwenklimitierung erfolgen. Die Erfindung umfasst daher besonders eine Gelenkanordnung zur wahlweisen Aufnahme unterschiedlich dimensionierter Limitierungskeile zum Zwecke der Wahl oder Anpassung der Schwenklimitierung und die Verwendung unterschiedlich dimensionierter Limitierungskeile zum Zwecke der Wahl oder Anpassung der Schwenklimitierung in der erfindungsgemäßen Gelenkanordnung.

In einer bevorzugten Ausgestaltung ist zur Erweiterung des Anwendungs- und Therapiespektrums der Gelenkorthese auch vorgesehen, dass in das Gelenkgehäuse zwei Limitierungskeile eingesetzt werden, wodurch die Schwenkbewegung der Gelenkschiene in beide Schwenkrichtungen begrenzbar ist. Dazu ist besonders vorgesehen, dass zumindest eine der Gehäuseplatten des Gelenkgehäuses zumindest zwei bevorzugt gegenüberliegende Durchbrechungen aufweist, in die jeweils in axialer Richtung ein Limitierungskeil einsetzbar ist. Auch in diesem Fall können beide Limitierungskeile einfach von derselben Seite in das Gehäuse eingesetzt werden. Bedienung und mechanische Wirkung eines Gelenks mit zwei einsetzbaren Limitierungskeilen unterscheiden sich dabei nicht von der hierin beschriebenen Ausgestaltung mit einem Limitierungskeil. Die hierin für eine Ausführung mit einem Limitierungskeil beschriebenen Vorteile und Wirkungen gelten demgemäß für die bevorzugte Ausführung mit zwei Limitierungskeilen entsprechend. Die Anordnung der Limitierungskeile zur Begrenzung der Schwenkbewegung der Gelenkschienen in beiden Schwenkrichtungen ergibt sich für den Fachmann in Kenntnis der hierin beschriebenen Ausführung mit einem einzigen einsetzbaren Limitierungskeil ohne weiteres. Bevorzugt wird dazu in der mindestens einen Gehäuseplatte eine zweite Durchbrechung vorgesehen, die der ersten Durchbrechung der Gehäuseplatte zur Aufnahme des ersten Limitierungskeils in der Schnittebene durch das Gelenk, welche im Wesentlichen in der Winkelhalbierenden des Verschwenkwinkels der Gelenkschienen liegt und parallel zu den Schwenkachsen und bevorzugt in den Schwenkachsen verläuft, gespiegelt gegenüber.

In einer besonderen Ausgestaltung ist zusätzlich mindestens ein Deckel vorgesehen, mit dem das Gelenkgehäuse abdeckbar ist. Bevorzugt ist, ausschließlich oder mindestens, das Gelenkgehäuse an der äußeren Seite, an der äußeren Gehäuseplatte, das heißt im angelegten Zustand der Gelenkorthese auf der körperabgewandten Seite, mit diesem Deckel abdeckbar. Dabei ist besonders vorgesehen, dass der Deckel an dem Gehäuse über mindestens einem Verschlussriegel oder ein vergleichbares Verschlusselement mit einem in dem Gehäuse, bevorzugt in den Gehäusedeckel ausgebildeten Gegenverschlusselement eingreifen kann, bevorzugt formschlüssig, um den Deckel an dem Gehäuse zu halten. Vorteilhafterweise deckt der Deckel die Gehäuseplatte zumindest im Bereich der Durchbrechung zur Aufnahme des Limitierungskeils in dem Gehäuse so ab, dass ein dort eingesetzter Limitierungskeil nicht aus dem Gehäuse ausrücken kann. Durch diese einfache Weise kann einer oder mehrere Limitierungskeile in dem Gelenkgehäuse sicher gehalten werden. Vorzugsweise sind keine weiteren Maßnahmen zur Sicherung des oder der Limitierungskeile in dem Gelenkgehäuse erforderlich. In bevorzugter Ausgestaltung dieses Deckels weist dieser zumindest ein Rastelement, bevorzugt eine Rastnase, auf, welche spezifisch ausgestaltet ist, um den mindestens einen Limitierungskeil an dem Deckel zu halten, sodass der Limitierungskeil zusammen mit dem Deckel kontrollierbar in dem mindestens eine Durchbrechung in der Gehäuseplatte einsetzbar oder herausnehmbar ist. Diese Ausgestaltung erlaubt besonders vorteilhaft, dass, besonders wenn die Gelenkorthese bereits am Körper getragen wird, der Limitierungskeil zusammen mit dem Deckel leicht aus dem Gelenk herausgenommen oder eingesetzt werden kann. Besonders in Verbindung mit verschieden dimensionierten Limitierungskeilen zur genauen Anpassung des Grades der Schwenklimitierung kann so auf einfache Weise in der praktischen Anwendung der passende Limitierungskeil aus einem "Baukastensystem" ausgewählt, in den erfindungsgemäßen Deckel eingesetzt, der dort bevorzugt an einer Rastnase einrastet und an dem Deckel gehalten wird, um anschließend direkt in das Gelenkgehäuse verbracht zu werden. Alternativ kann der Limitierungskeil auch direkt in das Gelenkgehäuse gesetzt und anschließend der Deckel aufgesetzt werden, der dabei bevorzugt den Limitierungskeil gegen ein Ausrücken oder Herausfallen aus der Durchbrechung schützt und in dem Gelenkgehäuse an Ort und Stelle hält. Dabei ist bevorzugt vorgesehen, dass beim Aufsetzen des Deckels die an dem Deckel vorzugsweise vorgesehene Rastnase auch den bereits in das Gelenkgehäuse eingesetzten Limitierungskeil greift und mit dem Deckel verbindet. Dabei kann besonders durch Abnehmen dieses Deckels auch leicht der axial eingesetzte Limitierungskeil mit aus dem Gelenkgehäuse genommen werden. In diesen Ausgestaltungen dient also der Gelenkdeckel als Werkzeug zum einfachen und kontrollierten Einsetzen und Entnehmen des Limitierungskeils. Vorteilhafterweise können alle diese Maßnahmen zum Einsetzen oder Austauschen des oder der Limitierungskeile auch leicht an einer bereits an den Patienten angelegten Gelenkorthese durchgeführt werden, so dass eine einfachere und sicherere Handhabung möglich ist.

Weiter besitzt der Deckel im Bereich des einsetzbaren Limitierungskeils in bevorzugter Ausgestaltung mindestens ein Fenster, wodurch ein Limitierungskeil auch im an das Gelenkgehäuse angebauten Zustand sichtbar bleibt. Eine in den Limitierungskeil eingeschlagene Zahl, besonders eine Gradzahl-Prägung, und/oder sonstige Markierung, besonders eine Farbmarkierung, von Außen sichtbar und dient der Identifizierung des gerade eingesetzten Limitierungskeils. Auch dabei ist die erfindungsgemäße axiale Einsetzrichtung des Limitierungskeils von Vorteil in der Anwendung, da die Markierung einfach auch bei seitlicher Ansicht der angelegten Orthese abgelesen werden kann.

Gegenstand der Erfindung ist auch eine Gelenkorthese, enthaltend zumindest ein biaxiales Gelenk, worin erfindungsgemäß in axialer Richtung mindestens ein Limitierungskeil einsetzbar ist. Besonderer Gegenstand der Erfindung ist eine Kniegelenksorthese mit mindestens einem, bevorzugt zweier dieser erfindungsgemäßen biaxialen Gelenke.

Schließlich betrifft die Erfindung auch die Verwendung mindestens eines in axialer Richtung in ein Gelenkgehäuse einsetzbaren Limitierungskeils zur Limitierung des Schwenkwinkels von Gelenkschienen in einem biaxialen Gelenk, wie es hierin beschrieben ist.

Die Erfindung wird durch die nachfolgenden spezifischen Ausführungsbeispiele und Figuren näher beschrieben, ohne dass diese beschränkend zu verstehen wären.

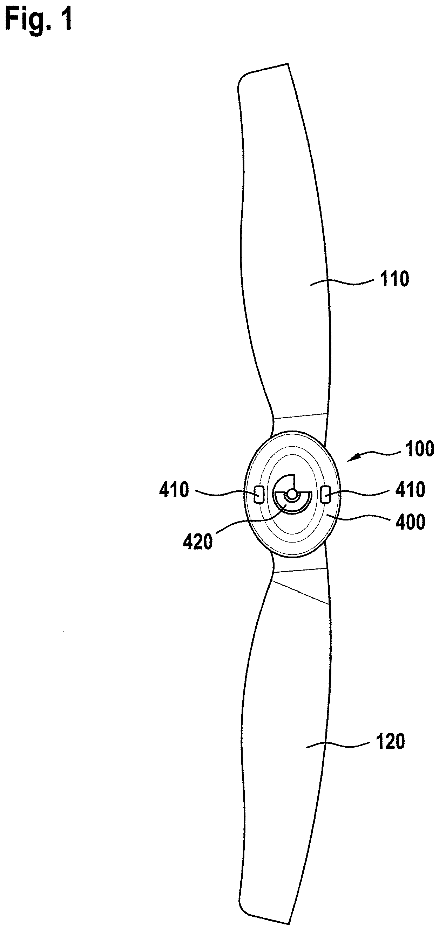

Figur 1 zeigt eine schematische Gesamtansicht in Draufsicht einer Ausführung der erfindungsgemäßen Gelenkschiene zur Anwendung in einer Gelenkorthese, besonders Hartrahmenorthese für das Kniegelenk. Von dem Gelenk 100 erstrecken sich die beiden darin gelenkig gelagerten armförmigen Gelenkschienen 110, 120. Das Gelenkgehäuse 200 (nicht sichtbar) ist in dieser Darstellung zumindest auf der im angelegten Zustand der Hartrahmenorthese von dem Körpergelenk abweisenden Seite des Gelenkgehäuses vollständig mit dem Deckel 400 abgedeckt. Der Deckel 400 weist in der dargestellten Ausführung mindestens ein Fenster 410 auf. Der Deckel 400 ist über einen zentralen Verschlussriegel 420 an einem in dem Gehäuse ausgebildeten Gegenverschlusselement 246 (nicht gezeigt) festgehalten.

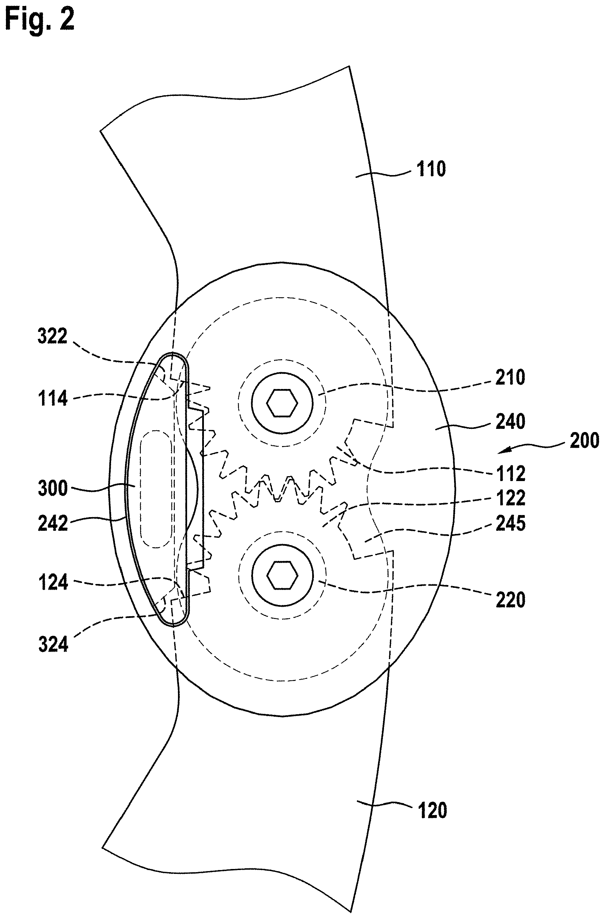

Figur 2 zeigt eine schematische Detailansicht in Draufsicht einer erfindungsgemäßen Ausführung des Gelenkgehäuses 200 mit Deckel 400 und Limitierungskeil 300. Die Gelenkschienen 110, 120 werden jeweils im Lagerzapfen 210, 220 gelagert und zwischen zwei Gehäuseplatten 240, 250 geführt. Gezeigt ist in dieser Ansicht nur die äußere Gehäuseplatte 240. Zweckmäßigerweise sind die Lagerzapfen 210, 220 mit beiden Gehäuseplatten 240, 250 verschraubt oder anderweitig fest verbunden und beabstanden die beiden Gehäuseplatten 240, 250 zueinander. Optional sind zwischen Gehäuseplatten und den Gelenkschienen jeweils Gleitplatten 245, 255 vorgesehen. In der Figur gezeigt ist die äußere Gleitplatte 245 zwischen den Gelenkschienen 110, 120 und der äußeren Gehäuseplatte 240. Die Gelenkköpfe 112, 122 der Gelenkschienen kämmen beim Verschwenken in einer Verzahnung. An den Gelenkköpfen 112, 122 sind jeweils Schultern 114, 124 ausgebildet, und zwar zumindest auf einer Seite im Anschluss an den dort jeweils ausgebildeten Zahnkranz. Zumindest auf dieser einer Seite des Gelenks ist zumindest in einer der Gehäuseplatten 240, 250 des Gelenkkörpers 200, bevorzugt zumindest in der äußeren Gehäuseplatte 240 eine Ausnehmung oder Durchbrechung 242 vorgesehen, die der Aufnahme eines in axialer Richtung einsetzbaren Limitierungskeils 300 dient. In bevorzugter Ausgestaltung ist zusätzlich in der inneren Gehäuseplatte 250 eine Durchbrechung 252 vorgesehen, welche mit einer Schulter desselben Limitierungskeils 300 in Eingriff kommen kann, um den Limitierungskeil sicher beidseits in dem Gelenkgehäuse 200 zu führen. Beim Verschwenken der Gelenkschienen 110, 120 kommen ab einem bestimmten Schwenkwinkel die jeweiligen Schultern 114, 124 mit Anschlagflächen 322, 324 des Limitierungskeils in Kontakt und begrenzen so die weitere Verschwenkung der Gelenkschienen 110, 120. Je nach Dimensionierung des Limitierungskeils 300, besonders der Beabstandung der Anschlagflächen 322, 324 zueinander, wird der Verschwenkwinkel der Gelenkschienen 110, 120 in dem Gelenk 100 mehr oder weniger stark limitiert. In einer Variante sind zusätzlich auch auf der gegenüberliegenden Seite der jeweiligen Gelenköpfe 112, 122, jeweils entsprechende Schultern 114, 124 ausgebildet. Der Deckel 400 ist über einen zentralen Verschlussriegel 420 an einem in dem Gelenkgehäuse 200 ausgebildeten Gegenverschlusselement 246 fixierbar.

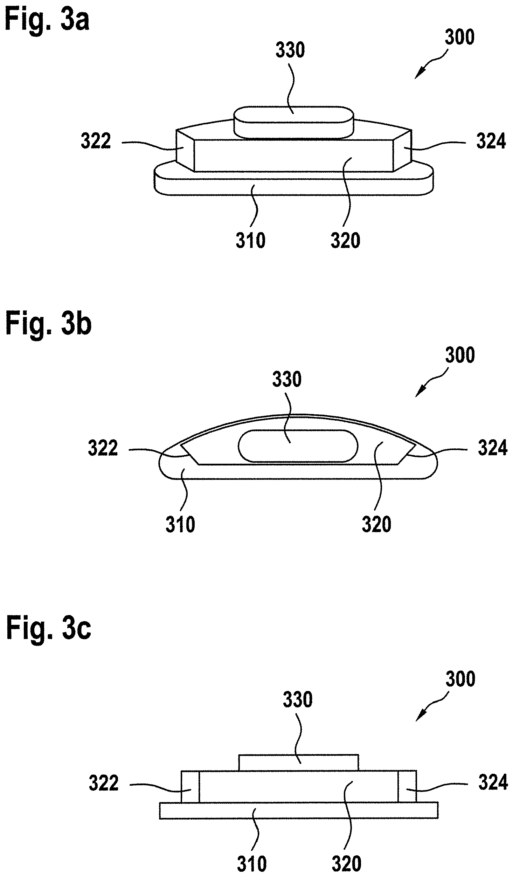

Die Figuren 3a, 3b, 3c zeigen Ansichten eines erfindungsgemäß in das Gelenkgehäuse einsetzbaren Limitierungskeils. Dieser teilt sich in zumindest drei funktionale Abschnitte ein. Eine äußere Führungsschulter 310 ist besonders ausgebildet, um mit der Durchbrechung 242 der äußeren Gehäuseplatte 240 in Eingriff zu kommen. Eine innere Führungsschulter 330 ist besonders ausgebildet, um mit der Durchbrechung 252 der inneren Gehäuseplatte 250 in Eingriff zu kommen. Der dazwischen liegende zentrale Abschnitt 320 dient der funktionalen Limitierung der Schwenkbewegung. Dazu weist dieser zentrale Limitierungsabschnitt 320 Anschlagsflächen 322, 324 auf, die im eingesetzten Zustand mit den an den Gelenkköpfen 112, 122 jeweils ausgebildeten Schultern 114, 124 in Kontakt kommen. Die Figuren 3a, 3b, 3c zeigen eine für die erfindungsgemäße axiale Einsetzrichtung besonders geeignete Ausgestaltung des Limitierungskeils 300. Andere Ausgestaltungen und Varianten davon, die ein axiales Einsetzen, besonders von der Seite der äußeren Gehäuseplatte 240 her ermöglichen und einen festen Sitz des Limitierungskeils 300 in dem Gelenkgehäuse 200 gewährleisten, sind ebenfalls denkbar und Gegenstand dieser Erfindung.

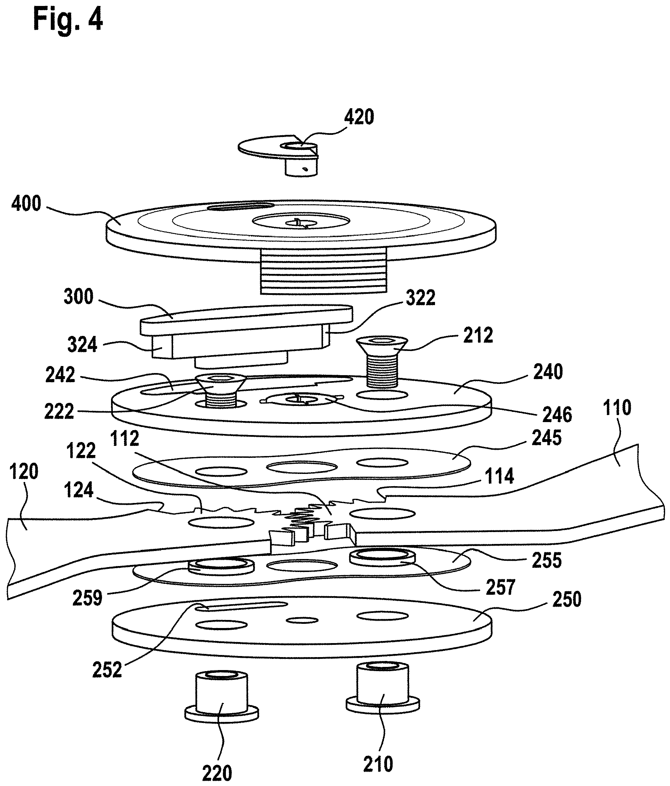

Figur 4 zeigt eine spezifische Ausgestaltung des erfindungsgemäßen Gelenks in Detailansicht in perspektivischer Explosionsdarstellung der Ausführung nach Figur 2. Die beiden Gehäuseplatten 240, 250 sind in dieser Ausgestaltung mittels der beiden Lagerzapfen 210, 220 über Schrauben 212, 222 miteinander fest verschraubt und voneinander beabstandet. Die Gelenkschienen 110, 120 werden an den Lagerzapfen 210, 220 jeweils schwenkbar gelagert. Besonders weisen die Gleitplatten 245, 255 jeweils Lagerschultern 257, 259 auf, die jeweils eine Gleitlagerhülse zwischen den Lagerzapfen 210, 220 und dem jeweiligen Gelenkauge der Gelenkschienen 110, 120 bilden.

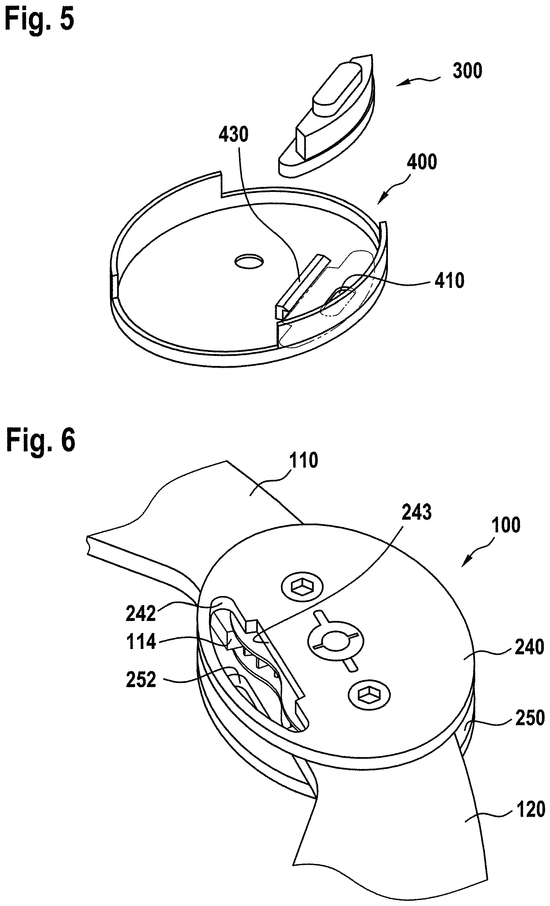

Figur 5 zeigt eine perspektivische Darstellung des Deckels 400 und den Limitierungskeil 300. In bevorzugter Ausgestaltung ist der Limitierungskeil 300 in den Deckel 400 einsetzbar und wird dort durch mindestens ein Rastelement 430, das an dem Deckel ausgebildet ist, gehalten. In dieser Ausführung ist der Limitierungskeil 300 zusammen mit dem Deckel 400 als Einheit in das Gelenkgehäuse 200 in der erfindungsgemäßen Weise einsetzbar und herausnehmbar. Dazu kann zumindest die Durchbrechung 242 der äußeren Gehäuseplatte 240 mindestens eine zusätzliche Ausnehmung 243 zur Aufnahme der Rastnase 430 des Deckels 400 im zusammengesetzten Zustand aufweisen. Die mindestens eine Ausnehmung 243 in der Durchbrechung 242 der äußeren Gehäuseplatte kann auch der Codierung der dort einsetzbaren Limitierungskeile dienen, um die Auswahlmöglichkeit beim Einsatz zu begrenzen. Die einsetzbaren Limitierungskeile weisen dazu selektiv an der äußeren Führungsschulter 310 zusätzliche Ausbuchtungen oder Nasen auf, die nach dem Schlüssel-Schloss-Prinzip mit der Ausnehmung 243 korrespondieren. Durch das Fenster 410 im Deckel kann ein auf der Außenfläche des Limitierungskeils 300, besonders in der äußeren Führungsschulter 310, vorhandener Aufdruck oder eine Gravur auch im zusammengesetzten Zustand des Gelenks erkannt und damit der jeweils eingesetzte Typ des Limitierungskeils angezeigt werden.
Figur 6 zeigt eine perspektivische Ansicht des Gelenks 100 nach Figur 2 und 3 mit entsprechenden Ausnehmungen 242, 243 zumindest in der äußeren Gehäuseplatte zur Aufnahme des Deckels 400 mit Limitierungskeil 300 nach Figur 5.

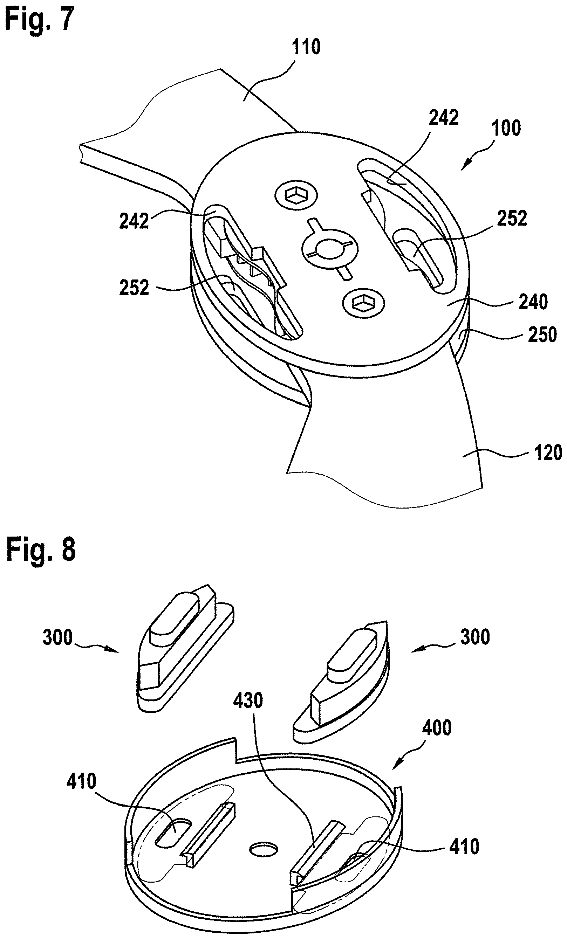

Figur 7 zeigt eine perspektivische Ansicht einer Variante des Gelenks 100 nach Figur 2, 3 und 6, wobei beiderseits der Gelenkschienen 110, 120 Durchbrechungen 242, 252 zur Aufnahme von Limitierungskeilen 300 auf gegenüberliegenden Seiten in dem Gelenkgehäuse 200 ausgebildet sind, um die Schwenkbewegung der Gelenkschienen 110, 120 in dem Gelenkgehäuse 200 in beide Schwenkrichtungen geeignet zu limitieren.
Figur 8 zeigt eine perspektivische Darstellung eines Deckels 400 und Limitierungskeile 300, spezifisch passend für die in Figur 7 dargestellte Variante mit zwei gegenüberliegenden Ausnehmungen 242 zur Aufnahme zweier Limitierungskeile 300. Besonders sind die Limitierungskeile 300 jeweils in den Deckel 400 einsetzbar und werden dort bevorzugt durch jeweils mindestens ein Rastelement 430, das an dem Deckel 400 ausgebildet ist, gehalten. Durch die Fenster 410 im Deckel kann jeweils ein auf der Außenfläche des Limitierungskeils 300, besonders in der äußeren Führungsschulter 310, vorhandener Aufdruck oder eine Gravur auch im zusammengesetzten Zustand des Gelenks erkannt und damit der jeweils eingesetzte Typ des Limitierungskeils angezeigt werden.

## Patentansprüche

1. Biaxiales Gelenk (100) für eine Gelenkorthese, enthaltend:
Gelenkschienen (110,120) mit jeweils gezahnten Gelenkköpfen (112,122), die in einem Gelenkgehäuse (200) zwischen äußerer und innerer Gehäuseplatten (240,250) geführt sind und an Gelenkachsen (210,220) in dem Gelenkgehäuse gelagert sind, wobei die Gelenkschienen (110,120) an ihren gezahnten Gelenkköpfen (112,122) jeweils miteinander kämmend gemeinsam um die Gelenkachsen (210,220) schwenkbar sind,
**dadurch gekennzeichnet, dass** mindestens eine der Gehäuseplatten (240,250) mindestens eine Durchbrechung (242,252) zur jeweiligen Aufnahme eines Limitierungskeils (300) in axialer Richtung aufweist, wobei der Limitierungskeil (300) im eingesetzten Zustand mit korrespondierenden Schultern (114,124) der Gelenkköpfe (112,122) ab einem bestimmten Verschwenkwinkel der Gelenkschienen (110,120) in Eingriff kommen kann, um die Verschwenkung der Gelenkschienen (110,120) in zumindest eine Schwenkrichtung zu begrenzen.

2. Gelenk nach Anspruch 1, wobei beide Gehäuseplatten (240,250) jeweils eine Durchbrechung (242,252) zur Aufnahme und Führung dieses Limitierungskeils (300) in axialer Richtung aufweisen.

3. Gelenk nach Anspruch 1 oder 2, wobei der einstückige Limitierungskeil (300) in zumindest drei funktionale Abschnitte einteilbar ist: eine äußere Führungsschulter (310), die mit der Durchbrechung (242) der äußeren Gehäuseplatte (240) in Eingriff kommen kann,
eine innere Führungsschulter (330), die mit der Durchbrechung (252) der inneren Gehäuseplatte (250) in Eingriff kommen kann und
einem dazwischenliegenden zentralen Limitierungsabschnitt (320) mit Anschlagsflächen (322,324).

4. Gelenk nach einem der vorstehenden Ansprüche, wobei das Gelenkgehäuse (200) mit zumindest einem Deckel (400) abdeckbar ist, wobei der Deckel (400) an dem Gehäuse (200) über einen Verschlussriegel (420), der in ein in dem Gehäuse (200) ausgebildeten Gegenverschlusselement (246) eingreifen kann, um den Deckel (400) an dem Gehäuse (200) zu halten.

5. Gelenk nach Anspruch 4, wobei der Deckel (400) zumindest ein Rastelement (430) aufweist, welches spezifisch ausgestaltet ist, um den Limitierungskeil (300) an dem Deckel (400) zu halten, sodass der Limitierungskeil (300) zusammen mit dem Deckel (400) kontrollierbar in die mindestens eine Durchbrechung (242,252) der Gehäuseplatten (240,250) einsetzbar oder herausnehmbar ist.

6. Gelenk nach einem der vorstehenden Ansprüche, wobei zwischen Gehäuseplatte (240,250) und den Gelenkschienen (110,120) jeweils eine Gleitplatte (245,255) eingesetzt ist.

7. Gelenk nach einem der vorstehenden Ansprüche, wobei in zumindest einer Gehäuseplatte (240,250) zwei in dieser Gehäuseplatte gegenüberliegende Durchbrechungen (242,252) ausgebildet sind, zur getrennten Aufnahme jeweils eines Limitierungskeils (300), um die Verschwenkung der Gelenkschienen (110,120) in beiden Schwenkrichtungen zu begrenzen.

8. Gelenkorthese, enthaltend zumindest ein biaxiales Gelenk nach einem der vorstehenden Ansprüche.

9. Gelenkorthese nach vorstehendem Anspruch, wobei die Gelenkorthese eine Kniegelenkorthese ist.

10. Verwendung mindestens eines in axialer Richtung in ein Gelenkgehäuse einsetzbaren Limitierungskeils zur Limitierung des Schwenkwinkels der Gelenkschienen in einem biaxialen Gelenk nach einem der Ansprüche 1 bis 7.

## Claims

1. A biaxial joint (100) for a joint orthosis, comprising:
hinge rails (110, 120) each having toothed joint heads (112, 122), the joint heads being guided in a joint housing (200) between outer and inner housing plates (240, 250) and being rotatable mounted in joint axes (210, 220) within the joint housing, the hinge rails (110, 120) being rotatable together about the joint axes (210, 220) with their toothed joint heads (112, 122) intermeshing with each other,
**characterized in that** at least one of the housing plates (240, 250) comprises at least one opening (242, 252) for the respective insertion of a limiting wedge (300) in axial direction, wherein the limiting wedge (300) in the inserted state can engage at a determined rotation angle with corresponding shoulders (114, 124) on the joint heads (112, 122) of the hinge rails (110; 120) to limit rotation of the hinge rails in at least one pivoting direction.

2. The joint according to claim 1, wherein both housing plates (240, 250) each have an opening (242, 252) for receiving and guiding said limiting wedge (300) in axial direction.

3. The joint according to claim 1 or 2, wherein the one-pieced limiting wedge (300) comprises at least three functional sections:
an outer guiding shoulder (310) engageable with the opening (242) of the outer housing plate (240);
an inner guiding shoulder (330) engageable with the opening (252) of the inner housing plate (250); and
an intermediate central limiting portion (320) having stop surfaces (322,324).

4. The joint according to any one of the preceding claims, wherein the joint housing (200) is coverable with at least one cover (400), the cover (400) on the housing (200) being engageable via a locking latch (420) with a mating locking element (246) formed within the housing (200) to hold the cover (400) to the housing (200).

5. The joint according to claim 4, wherein the cover (400) has at least one latching element (430) specifically designed to hold the limiting wedge (300) at the cover (400) so that the limiting wedge (300) together with the cover (400) is controllably insertable or removable at the at least one opening (242, 252) of the housing plates (240, 250).

6. The joint according to any one of the preceding claims, wherein a sliding plate (245, 255) is inserted between each housing plate (240, 250) and the hinge rails (110, 120).

7. The joint according to any one of the preceding claims, wherein in at least one housing plate (240, 250) two opposite openings (242, 252) are formed, for separately receiving in each case a limiting wedge (300) to limit rotation of the hinge rails (110, 120) in both pivoting directions.

8. A joint orthosis, comprising at least one biaxial joint according to any one of the preceding claims.

9. A joint orthosis according to the preceding claim, wherein the joint orthosis is a knee orthosis.

10. Use of at least one limiting wedge, which is insertable in a joint housing in the axial direction, for limiting the rotation angle of hinge rails of a biaxial joint according to any one of claims 1 to 7.

## Revendications

1. Articulation biaxiale (100) pour une orthèse articulée, contenant :
des rails d'articulation (110, 120) dotés respectivement de têtes d'articulation dentées (112, 122), qui sont guidés dans un boîtier d'articulation (200) entre des plaques de boîtier intérieure et extérieure (240, 250) et sont disposés aux axes d'articulation (210, 220) dans le boîtier d'articulation, dans laquelle les rails d'articulation (110, 120) peuvent pivoter ensemble, à leurs têtes d'articulation dentées (112, 222) engrenées l'une avec l'autre, autour des axes d'articulation,
**caractérisée en ce qu'**au moins une des plaques de boîtier (240, 250) comporte au moins une découpe (242, 252) dans la direction axiale pour recevoir respectivement une cale de limitation (300), dans laquelle la cale de limitation (300) peut, à l'état monté, venir en prise avec des épaulements (114, 124) correspondants des têtes d'articulation (112, 122) à un angle de pivot prédéterminé des rails d'articulation (110,120), afin de limiter le pivotement des rails d'articulation (110, 120) dans au moins une direction de pivot.

2. Articulation selon la revendication 1, dans laquelle les deux plaques de boîtier (240, 250) comprennent respectivement une découpe (242, 252) pour recevoir et guider cette cale de limitation dans la direction axiale.

3. Articulation selon la revendication 1 ou 2, dans laquelle la cale de limitation (300) en une pièce peut être partagée en au moins trois sections fonctionnelles :
un épaulement de guidage externe (310), pouvant venir en prise avec la découpe (242) de la plaque de boîtier externe (240),
un épaulement de guidage interne (330), pouvant venir en prise avec la découpe (252) de la plaque de boîtier interne (250) et
une section de limitation centrale (320) se trouvant entre ceux-ci, dotée de surfaces de butée (322, 324).

4. Articulation selon l'une des revendications précédentes, dans laquelle le boîtier d'articulation (200) peut être recouvert par au moins un capot (400), dans laquelle le capot (400) est fixé au boîtier (200) par un verrou (420), pouvant venir en prise avec un élément de contre-verrou (246) réalisé dans le boîtier (200), afin de maintenir le capot (400) sur le boîtier (200).

5. Articulation selon la revendication 4, dans laquelle le capot (400) comporte au moins un élément de cliquet (430), lequel est spécifiquement conçu pour maintenir la cale de limitation (300) sur le capot (400), de sorte que la cale de limitation (300) peut être insérée en même temps que le capot (400) dans l'au moins une découpe (242, 252) des plaques de boîtier (240, 250) ou en être détaché de manière contrôlable.

6. Articulation selon l'une des revendications précédentes, dans laquelle entre les plaques de boîtier (240, 250) et les rails d'articulation (110, 120) est respectivement insérée une plaque coulissante (245, 255).

7. Articulation selon l'une des revendications précédentes, dans laquelle dans au moins une plaque de boîtier (240, 250) deux découpes (242, 252) opposées l'une à l'autre sont réalisées dans cette plaque, afin de recevoir séparément respectivement une cale de limitation (300), afin de délimiter le mouvement de pivot des rails d'articulation (110, 120) dans les deux directions de pivot.

8. Orthèse articulée, comprenant au moins une articulation biaxiale selon l'une des revendications précédentes.

9. Orthèse articulée selon la revendication précédente, dans laquelle l'orthèse articulée est une orthèse articulée de genou.

10. Utilisation d'au moins une cale de limitation insérable dans un boîtier d'articulation dans la direction axiale afin de limiter l'angle de pivot des rails d'articulation dans une articulation biaxiale selon l'une des revendications 1 à 7.
